Europäisches Patentamt

European Patent Office

Office européen des brevets

(11) Veröffentlichungsnummer : **0 017 945
B1**

# EUROPÄISCHE PATENTSCHRIFT

(45) Veröffentlichungstag der Patentschrift :
17.03.82

(51) Int. Cl.³ : **C 07 D201/04**

(21) Anmeldenummer : **80101986.0**

(22) Anmeldetag : **14.04.80**

(54) **Verfahren zur Herstellung von Caprolactam.**

(30) Priorität : **14.04.79 DE 2915360**

(43) Veröffentlichungstag der Anmeldung :
**29.10.80 (Patentblatt 80/22)**

(45) Bekanntmachung des Hinweises auf die Patenterteilung : **17.03.82 Patentblatt 82/11**

(84) Benannte Vertragsstaaten :
**BE CH DE FR GB IT LI NL**

(56) Entgegenhaltungen :
**DE - B - 1 195 318
DE - A - 2 237 170
DE - A - 2 837 793
FR - A - 1 329 135
FR - A - 1 451 825
FR - A - 1 519 552
FR - A - 2 364 895**

(73) Patentinhaber : **BASF Aktiengesellschaft
Carl-Bosch-Strasse 38
D-6700 Ludwigshafen (DE)**

(72) Erfinder : **Werther, Joachim, Dr.
An der Tuchbleiche 2
D-6712 Bobenheim-Roxheim 2 (DE)**
Erfinder : **Fuchs, Hugo, Dr.
Egellstrasse 28
D-6700 Ludwigshafen (DE)**
Erfinder : **Brand, Uwe
Rheingoldstrasse 12
D-6841 Rosengarten (DE)**
Erfinder : **Faulhaber, Friedrich Richard, Dr.
Ostpreussenstrasse 3
D-6704 Mutterstadt (DE)**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Imprimerie Jouve, 18, rue St-Denis, 75001 Paris, France

Verfahren zur Herstellung von Caprolactam

Gegenstand der Erfindung ist ein Verfahren zur Herstellung von Caprolactam, bei dem man in einer ersten Stufe Cyclohexanonoxim unter Mitverwendung von Inertgasen verdampft, das Gemisch aus dampfförmigem Cyclohexanonoxim und Inertgasen in eine zweite Stufe leitet und dort bei einer Temperatur von 230 bis 450 °C an Bortrioxid enthaltenden Trägerkatalysatoren in der Wirbelschicht Cyclohexanonoxim in Caprolactam umlagert.

In der DE-PS 1 195 318 ist ein Verfahren zur katalytischen Umlagerung von Cyclohexanonoxim beschrieben, bei dem man flüssiges Cyclohexanonoxim in ein Wirbelbett aus saurem Katalysator leitet, das Cyclohexanonoxim verdampft und gleichzeitig umlagert. Es läßt sich jedoch nicht vermeiden, daß sich Cyclohexanonoxim im gewissen Maße zersetzt und Crackprodukte bildet, die sich auf dem Katalysator ablagern und diesen schädigen. Darüberhinaus wird das erzeugte Caprolactam durch schwer zu beseitigende Verunreinigungen in seiner Qualität gemindert. Solche Verunreinigungen verursachen insbesondere eine erhöhte UV-Kennzahl sowie eine erhöhte Permanganat Titrationszahl. Es bedarf eines erheblichen technischen Aufwandes, um Reincaprolactam zu erhalten, das den Anforderungen hinsichtlich der UV-Kennzahl und der Permanganat Titrationszahl genügt. Aus der DE-PS 1 195 318 ist auch bereits bekannt, Cyclohexanonoxim in einem Fallfilmverdampfer zunächst zu verdampfen und dempfförmiges Cyclohexanonoxim in einer zweiten Stufe umzulagern. Es wird jedoch darauf hingewiesen, daß die gesonderte Verdampfung am Fallfilmverdampfer nicht zum Erfolg führt. Ferner wird in der DE-OS 2 641 414 ein Verfahren beschrieben, bei dem man Cyclohexanonoxim unter bestimmten Druck- und Temperaturbedingungen an Fallfilmverdampfern verdampft. Hierbei müssen jedoch erhebliche Mengen an Cyclohexanonoxim bei hoher Temperatur umgewälzt werden. Ferner ist es aufwendig, die Verdampfungsgeschwindigkeit den betrieblichen Anforderungen anzupassen.

Es war deshalb die technische Aufgabe gestellt, die katalytische Umlagerung von Cyclohexanonoxim so zu gestalten, daß die Zersetzung von Cyclohexanonoxim und die Bildung von schwer zu beseitigenden Verunreinigen auf ein Minimum reduziert wird.

Diese technische Aufgabe wird gelöst in einem Verfahren zur Herstellung von Caprolactam, bei dem man in einer ersten Stufe Cyclohexanonoxim unter Mitverwendung von Inertgasen verdampft, das Gemisch aus dampfförmigem Cyclohexanonoxim und Inertgasen in eine zweite Stufe leitet und dort bei einer Temperatur von 230 bis 450 °C an Bortrioxid enthaltenden Trägerkatalysatoren in der Wirbelschicht Cyclohexanonoxim in Caprolactam umlagert, wobei man das Cyclohexanonoxim in der ersten Stufe durch in Berührung bringen mit einer Wirbelschicht aus inerten Feststoffpartikeln bei einer Temperatur von 150 bis 250 °C verdampft.

Das neue Verfahren hat den Vorteil, daß das so erzeugte Caprolactam eine verbesserte UV-Kennzahl und Permanganat Titrationszahl aufweist und somit die Reinigung vereinfacht wird. Ferner müssen keine großen Mengen an Cyclohexanonoxim bei hoher Temperatur umgewälzt werden und die zu verdampfende Menge an Cyclohexanonoxim läßt sich leicht regeln und an Änderungen in der Umlagerungsstufe anpassen. Schließlich wird nach dem neuen Verfahren der Katalysator für die Umlagerung weniger geschädigt.

Erfindungsgemäß wird Cyclohexanonoxim in einer ersten Stufe durch in Berührung bringen mit einer Wirbelschicht aus inerten Feststoffpartikeln verdampft. Geeignete Feststoffpartikel sind beispielsweise Glaskugeln, Aluminiumoxid in seine verschiedenen Modifikationen wie Tonerde γ-Aluminiumoxid oder Böhmit, Kieselsäure, Titandioxid oder Gemische der genannten Oxide untereinander, z.B. Aluminiumsilikate ferner Quarzsand. Als besonders geeignet hat sich Quarzsand erwiesen. Die Feststoffpartikel haben vorteilhaft eine mittlere Korngröße von 0,02 bis 1,0 mm. Als Inertgase verwendet man vorzugsweise Stickstoff oder Kohlendioxid, insbesondere Stickstoff. Je Kilogramm zu verdampfendes Cyclohexanonoxim wendet man vorteilhaft 0,1 bis 10 kg Inertgase an. Es hat sich als zweckmäßig erwiesen, die Inertgase vor Eintritt in die Wirbelschicht auf 50 bis 250 °C vorzuwärmen. Vorzugsweise beträgt die Verweilzeit des Cyclohexanonoxims beim Verdampfen in der Wirbelschicht 0,01 bis 30 sec. Es hat sich ferner bewährt, die in der Umlagerungsstufe freiwerdende Wärme in der Verdampfungsstufe zu verwenden. Dies wird beispielsweise erzielt, indem man mittels eines Wärmeträgers über Kühlschlangen in der Umlagerungsstufe die Wärme abführt und mit dem heißen Wärmeträger die Feststoffpartikel in der Verdampfungsstufe beheizt. Die Verdampfung in der Wirbelschicht wird bei einer Temperatur von 150 bis 250 °C insbesondere 180 bis 220 °C durchgeführt. Es ist möglich, das zu verdampfende Cyclohexanonoxim in fester Form oder vorteilhaft flüssig in die Wirbelschicht einzubringen. Das verwendete Cyclohexanonoxim hat vorteilhaft einen Wassergehalt von 1 bis 10, insbesondere 3 bis 7 Gew.-%. Zweckmäßig wird das zu verdampfende Cyclohexanonoxim mit einem Teil der Inertgase in die Wirbelschicht eingedüst. Erfindungsgemäß wird somit Cyclohexanonoxim in dem Maße verdampft, wie es der Wirbelschicht zugeleitet wird.

Das aus der Wirbelschicht in der ersten Stufe entweichende Gemisch aus dampfförmigem Cyclohexanonoxim und Inertgasen, das zudem noch geringe Mengen an Wasser enthalten kann, entsprechend dem Wassergehalt des verwendeten Cyclohexanonoxims, wird in eine zweite Stufe geleitet und dort bei einer Temperatur von 230 bis 450 °C, insbesondere 270 bis 370 °C an Bortrioxid enthaltenden Trägerkatalysatoren in der Wirbelschicht Cyclohexanonoxim in Caprolactam umgelagert. Die Umlagerung kann man wie die Verdampfung des Cyclohexanonoxims in der ersten Stufe bei vermindertem bis leichtem Überdruck ausführen.

Als Katalysator verwendet man bekannte Katalysatoren bei denen Bortrioxid oder Borsäure, die unter Reaktionsbedingungen in Bortrioxid übergeht, auf Träger aufgebracht sind. Als Träger sind insbesondere Aluminiumoxid in seinen verschiedenen Modifikationen wie Tonerde, γ-Aluminiumoxid, Boehmit, ferner Kieselsäure oder Titandioxid oder Gemische derartiger Oxide, ferner Verbindungen der Oxide untereinander, z.B. Aluminiumsilikate, geeignet. Das Gewichtsverhältnis von Bortrioxid zu Träger beträgt im allgemeinen von 1 : 9 bis 1 : 1. Bei bevorzugt verwendeten Katalysatoren beträgt der Anteil an Bortrioxid 25 bis 50 Gew.-%. Die Katalysatoren können außerdem durch Zusätze z.B. von Mangan, Kobalt oder Nickel in einer Menge bis zu 10 Gew.-%, bezogen auf Bortrioxid und berechnet als Metall modifiziert sein. Der Zusatz erfolgt bei der Herstellung in Form von Salzen, die beim Erhitzen in die entsprechenden Oxide übergehen, wie Nitrate oder fettsaure Salze. Dementsprechend liegen die Metalle im fertigen Katalysator als Oxide oder deren Verbindungen mit Bortrioxid vor. Die Katalysatoren werden in üblicher Weise hergestellt, z.B. werden die Träger mit Borsäure oder Ammoniumboratlösung getränkt, bei 50 bis 500 °C getrocknet und anschließend zur Umwandlung der aufgebrachten Verbindungen in die entsprechenden Mischphasen mit Bortrioxid bei 600 bis 1 200 °C geglüht. Die Katalysatoren werden in üblicher Weise geformt, z.B. durch Anteigen von Bortrioxid und Träger mit wenig Wasser, vermengen im Kneter, Pressen der Maße zu Strängen oder Pillen, Trocknen und Glühen bei den angegebenen Temperaturen. Zweckmäßig verwendet man Korngrößen von 0,05 bis 1,5 mm, insbesondere von 0,2 bis 1,0 mm. Die Höhe der Katalysatorschicht wird vorteilhaft so gewählt, daß man eine Verweilzeit des Cyclohexanonoxims an der Katalysatorschicht von 0,01 bis 30 sec., insbesondere 0,1 bis 5 sec. erzielt.

Aus dem erhaltenen caprolactamhaltigen Gas wird Caprolactam vorteilhaft durch Abschrecken mit Caprolactam oder stufenweise zunächst durch Abschrecken mit Caprolactam und dann mit Wasser in einer Kolonne abgeschieden. Das abgetrennte Inertgas wird nach Abscheiden von Wasser zweckmäßig wieder der ersten Stufe zur Verdampfung des Cyclohexanonoxims zugeführt.

Caprolactam, das nach dem Verfahren der Erfindung hergestellt wird, eignet sich zur Herstellung von Polycaprolactam.

Das Verfahren nach der Erfindung sei in folgenden Beispielen veranschaulicht.

Beispiel

In einem senkrecht stehenden elektrisch beheizten und mit einem Lochboden versehenen Rohr von 1 000 mm Länge und 100 mm Durchmesser, werden 3 kg eines Quarzsandes der Korngröße 0,1 bis 0,5 mm mit 3 500 Nl/h Stickstoff gewirbelt. Die Temperatur im wirbelnden Sand beträgt ca. 250 °C. Über eine Düse werden stündlich 5,5 kg flüssiges Cyclohexanonoxim mit einen Wassergehalt von 4,2 % mit Hilfe eines Stickstoffstroms von 1 500 l/h in die Wirbelschicht eingebracht und verdampft. Das entstehende Dampfgemisch wird nach Passieren eines Zyklons zur Abscheidung von Staubteilchen über einen Lochboden in einen zweiten Reaktor der Abmessungen 2 000 mm Länge und 100 mm Durchmesser geleitet. Der Reaktor enthält 1 200 g eines Aluminiumoxid-Bortrioxidkatalysators der Zusammensetzung 53 % Aluminiumoxid, 47 % Bortrioxid und der Korngröße 0,2 bis 1,0 mm. Dieser Reaktor hat eine Temperatur von 350 °C. In der Wirbelschicht befindet sich eine Kühlschlange die mit einer zweiten Kühlschlange in der Sandwirbelschicht des Verdampfers verbunden ist. Zwischen beiden Kühlschlangen wird ein Ölkreislauf aufrecht erhalten. Die bei der Umlagerung frei werdende Wärme wird so abgeführt und bei der Verdampfung des Oxims wieder eingesetzt. Dem Reaktor werden stündlich 2,5 kg Katalysator entnommen und die gleiche Menge an frischem Katalysator zugeführt. Das aus dem Reaktor entweichende Dämpfegemisch wird in eine Glockenbodenkolonne geleitet. Hierbei werden die leichtersiedenden Bestandteile über Kopf der Kolonne geführt, während das Rohcaprolactam kondensiert wird. Das erhaltene Rohlactam weist eine Permanganattitrationszahl von 3 000 und eine UV-Zahl von 4 800 auf. Die Ausbeute an Caprolactam nach einer Destillation unter vermindertem Druck beträgt 95,1 % bezogen auf eingesetztes wasserfreies Cyclohexanonoxim.

Der ausgeschleuste Katalysator weist einen Abbrand von 3,3 % auf wenn dieser bei 800 °C unter Luftzutritt 5 Std. behandelt wird.

Vergleichsbeispiel

In einem senkrecht stehenden elektrisch beheizten Rohr der Länge 2 000 mm und einem Durchmesser von 100 mm welches am unteren Ende mit einem Lochboden versehen ist, werden 1 200 g des in Biespiel 1 beschriebenen Bortrioxid-Katalysators eingebracht und auf 350 °C erhitzt. Durch Einblasen von 3 500 Nl/h Stickstoff wird dieser zum Wirbeln gebracht. Über eine Düse werden mit Hilfe eines Stickstoffstroms von 1 500 Nl/h 5,5 kg Cyclohexanonoxim mit einem Wassergehalt von 4,2 % eingebracht und umgelagert. Die Umlagerungswärme wird über eine in der Wirbelschicht befindliche Kühlschlange, welche mit Öl gefüllt und über einen Thermostaten verbunden ist, abgeführt. Gleichzeitig werden stündlich 2,5 kg Katalysator durch den Reaktor geschleust. Das den Reaktor verlassende Dampfgemisch wird in eine Glockenbodenkolonne geleitet. Hierbei werden wie in Beispiel 1 beschrieben die leichtsiedenden Bestandteile über Kopf der Kolonne geführt und das Rohlactam kondensiert.

Das erhaltene Rohlactam weist eine Permanganattitrationszahl von 6 000 und eine UV-Zahl von 8 500 auf. Die Ausbeute an Caprolactam nach einer Destillation unter vermindertem Druck beträgt 94.0 % der Theorie bezogen auf eingesetztes wasserfreies Cyclohexanonoxim.

Der Abbrand des ausgeschleusten Katalysators beträgt 5,5 % wenn dieser bei 800 °C unter Luftzutritt 5 Std. behandelt wird.

### Ansprüche

1. Verfahren zur Herstellung von Caprolactam, bei dem man in einer ersten Stufe Cyclohexanonoxim unter Mitverwendung von Inertgasen verdampft, das Gemisch aus dampfförmigem Cyclohexanonoxim und Inertgasen in eine zweite Stufe leitet und dort bei einer Temperatur von 230 bis 450 °C an Bortrioxid enthaltenden Trägerkatalysatoren in der Wirbelschicht Cyclohexanonoxim in Caprolactam umlagert, dadurch gekennzeichnet, daß man das Cyclohexanonoxim in der ersten Stufe durch in Berührung bringen mit einer Wirbelschicht aus inerten Feststoffpartikeln bei einer Temperatur von 150 bis 250 °C verdampft.

2. Verfahren nach Anspruch 1, dadurch gekennzeichnet, daß man je Kilogramm zu verdampfendes Cyclohexanonoxim 0,1 bis 10 kg Inertgas anwendet.

3. Verfahren nach den Ansprüchen 1 und 2, dadurch gekennzeichnet, daß man die bei der Umlagerung in der zweiten Stufe freiwerdende Wärme für die Verdampfung des Cyclohexanonoxims in der ersten Stufe verwendet.

### Claims

1. A process for the preparation of caprolactam, wherein in a first stage, cyclohexanone-oxime is vaporized, in the presence of inert gases, and the mixture of cyclohexanone-oxime vapor and inert gases is passed into a second stage where the cyclohexanone-oxime is rearranged to caprolactam at from 230 to 450 °C over a supported catalyst, containing boron trioxide, in a fluidized bed, characterized in that the cyclohexanone-oxime in the first stage is vaporized by bringing it into contact with a fluidized bed of inert solid particles at from 150 to 250 °C.

2. A process as claimed in claim 1, characterized in that from 0.1 to 10 kg of inert gas are used per kilogram of cyclohexanone-oxime to be vaporized.

3. A process as claimed in claims 1 and 2, characterized in that the heat liberated during the rearrangement in the second stage is used for the vaporization of the cyclohexanone-oxime in the first stage.

### Revendications

1. Procédé de préparation de caprolactame, dans lequel on vaporise, dans un premier stade, du cyclohexanonoxime avec emploi simultané de gaz inertes, on dirige dans un deuxième stade, le mélange de cyclohexanonoxime vaporisé et de gaz inertes pour y transposer, à une température de 230 à 450 °C, sur des catalyseurs à support contenant du trioxyde de bore, en couche turbulente, le cyclohexanonoxime en caprolactame, caractérisé par le fait que l'on vaporise le cyclohexanonoxime, dans le premier stade, par amenée en contact avec une couche turbulente de particules solides inertes, à une température de 150 à 250 °C.

2. Procédé selon la revendication 1, caractérisé par le fait que l'on utilise 0,1 à 10 kg de gaz inerte par kilogramme de cyclohexanonoxime à vaporiser.

3. Procédé selon les revendications 1 et 2, caractérisé par le fait que l'on utilise, pour la vaporisation du cyclohexanonoxime dans le premier stade, la chaleur libérée lors de la transposition dans le deuxième stade.